# EUROPEAN PATENT APPLICATION

(11) **EP 3 928 777 A1**
(43) Date of publication of application: **29.12.2021**
(21) Application number: 21181517.0
(22) Date of filing: 24.06.2021
(51) Int. Cl.: A61K 31/421, A61P 25/32

(54) **4,5-DIHYDRO-OXAZOL-2-YLAMINE DERIVATIVES FOR USE IN THE PREVENTION OR TREATMENT OF ALCOHOL USE DISORDER**

(30) Priority: 25.06.2020 PL 43444920
(71) Applicant: Instytut Biologii Doswiadczalnej im. Marcelego Nenckiego Polskiej Akademii Nauk, 02-093 Warszawa (PL)
(72) Inventor: FRYCZ, Bartosz, 60-149 Poznan (PL); STEFANIUK, Marzena, 04-549 Warszawa (PL); NOWICKA, Klaudia, 02-348 Warszawa (PL); KACZMAREK, Leszek, 05-806 Komorow (PL)
(74) Representative: Patpol Kancelaria Patentowa Sp. z o.o.

(57) **Abstract**

The invention relates to 4,5-dihydro-oxazol-2-ylamine derivatives of general Formula I: 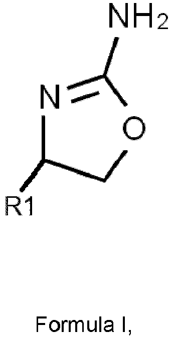 wherein R1 denotes:
- straight or branched (C1-C4) alkyl, optionally substituted, including in particular (C1-C4) alkyl substituted with a phenyl group, or
- phenyl, optionally substituted with one or more halogen, or
- 2-[(N-(C1-C2)alkyl-N-aryl)amin-(C1-C2)alkyl]- group
for use in a method of treating or preventing alcohol use disorder by reducing alcohol consumption and/or motivation to drink alcohol, wherein the compound of Formula I is administered prior to intended alcohol consumption to reduce motivation to drink alcohol.

## Description

### FIELD OF INVENTION

The invention relates to 4,5-dihydro-oxazol-2-ylamine derivatives for the use in the prevention or treatment of alcohol use disorder.

### BACKGROUND OF THE INVENTION

Alcohol addiction, also known as alcoholism, is a central nervous system disorder characterized among other by loss of control over the amount and frequency of alcohol consumption. Alcoholism development is related to, inter alia, abnormal dopamine secretion, which is the main neurotransmitter in the brain's reward system. Dopamine is responsible for individual satisfaction and directing motivational activities.

Pharmacological treatment of alcoholism is very difficult, and currently used drugs often do not bring desired results. Moreover, they are ineffective in preventing alcohol relapse. Several drugs intended for alcoholism treatment are available on the market (opioid antagonists Naltrexone and Nalmefene, Acamprosate with a possible NMDA receptor antagonist activity and Disulfiram inhibiting aldehyde dehydrogenase and enhancing the unpleasant effects of alcohol), but their effectiveness is still unsatisfactory.

Trace amine-associated receptor 1 (TAAR1) may play a key role in the regulation of dopamine secretion, and thus participate in the development of behavior related to excessive alcohol consumption.

Trace amine-associated receptor 1 (TAAR1) was identified in 2001 by two independent research teams. It was then noticed that TAAR1 is activated by some psychoactive compounds from the amphetamines group, and thus may participate in the development of addiction to these substances. First experiments with mice lacking the gene encoding said receptor provided evidence for the involvement of TAAR1 in the regulation of dopaminergic transmission, the disturbances of which underlie the development of compulsive behaviors characteristic of addiction. However, the real breakthrough in understanding the relationship between TAAR1 and the regulation of behavior related to psychoactive substances addiction was the development of selective agonists of this receptor by Roche in recent years. Some of these substances, including RO5256390, have been shown to be effective in suppressing compulsive behavior in cocaine-dependent or binge eating animals. However, the molecular mechanisms underlying the activity of these compounds are very poorly understood, which does not allow their commercial use yet. So far, only one study has been conducted to explain the role of TAAR1 in alcoholism development. In this study, animals lacking the TAAR1 gene and wild-type animals consumed alcohol in a standard two-bottle selection test. Animals without the TAAR1 gene tended to drink more alcohol. Interestingly, the mentioned differences between animal without the TAAR1 gene and wild-type, were statistically significant only for the lower alcohol percentages, but not for the higher concentrations used (9 and 11%) (Lynch et al., Trace Amine Associated Receptor 1 Modulates Behavioral Effects of Ethanol. Subst Abuse, 2013; 7: 117-26) compared to wild-type animals.

However, the influence of TAAR1 agonists on drinking behavior, including motivation to drink, has never been studied. The structural similarity of trace amines, which are endogenous ligands of TAAR1, to some stimulants from the amphetamines group, suggests a possible role of this receptor in the development of addiction to psychostimulants. Already at the time of the discovery of TAAR1, Bunzow et al., (Amphetamine, 3,4-methylenedioxymethamphetamine, lysergic acid diethylamide, and metabolites of the catecholamine neurotransmitters are agonists of a rat trace amine receptor. Mol Pharmacol. 2001; 60: 1181-1188) noticed that the receptor can be activated by direct action of amphetamine and 3,4-methylenedioxymethamphetamine. Activation of TAAR1 by amphetamine leads to a cascade of events that result in impaired dopaminergic transmission (Asif-Malik et al., Interaction Between the Trace Amine-Associated Receptor 1 and the Dopamine D2 Receptor Controls Cocaine's Neurochemical Actions. Sci Rep, 2017; 7 (1): 13901), which in turn underlies the motivations behind the exploration and abuse of a substance. Therefore, TAAR1 is a widely studied receptor in the context of the development of psychostimulant addiction (Liu et al., TAAR1 and Psychostimulant Addiction. Cell Mol Neurobiol. 2020; 40(2):229-238). Dopaminergic transmission disorders also occur during the development of alcoholism, however, compared to psychostimulant addiction, dopamine does not play a key role in this process (Gilpin and Koob, Neurobiology of Alcohol Dependence: Focus on Motivational Mechanisms. Alcohol Res Health. 2008; 31 (3): 185-95). Thus, the most attention, in view of the different neuronal mechanisms underlying the formation of these addictions, is paid to the role of TAAR1 in the development of psychostimulant addiction.

The present invention relates to 4,5-dihydro-oxazol-2-ylamine derivatives of general Formula I: 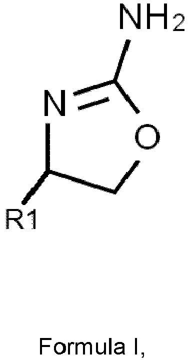 wherein R1 denotes:
- straight or branched (C1-C4) alkyl, optionally substituted, including in particular (C1-C4) alkyl substituted with a phenyl group, or
- phenyl, optionally substituted with one or more halogen, or
- 2-[(N-(C1-C2)alkyl-N-aryl)amine-(C1-C2)alkyl]- group
for use in a method of treating or preventing alcohol use disorder by reducing alcohol consumption and/or motivation to drink alcohol, wherein the compound of Formula I is administered prior to intended alcohol consumption to reduce motivation to drink alcohol.

The compounds for use in the present invention are selective agonists of the TAAR1 receptor. Exemplary compounds in this group include those commercially available (e.g., Sigma Aldrich):
- RO5256390 (nr CAS: 1043495-96-0) - ((S)-4-((S)-2-phenyl-butyl)-4,5-dihydro-oxazol-2-ylamine;
- RO5166017 (nr CAS: 1048346-74-2) - (4S)-4-[(N-ethylanilin)methyl]-4,5-dihydro-1,3-oxazol-2-ylamine;
- RO5203648 (nr CAS: 1043491-54-8) - (S)-4-(3,4-dichloro-phenyl)-4,5-dihydro-oxazol-2-ylamine;
Particularly preferably, the compound of general Formula I is a compound of Formula II:

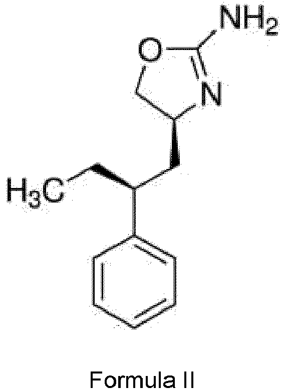

Consequently, the invention particularly relates to a compound of Formula II for use in a method of treating or preventing alcohol use disorder by reducing alcohol consumption and/or motivation to drink alcohol.

The compound of Formula II, i.e. ((S)-4-((S)-2-phenyl-butyl)-4,5-dihydro-oxazol-2-ylamine (CAS No.: 1043495-96-0), is a commercially available (Sigma-Aldrich; under the name RO5256390), selective agonist of trace amine-associated receptor 1.

TAAR1 agonists, including those described by general Formula I, and preparation methods thereof, have been described in the art (see Galley et al., Discovery and Characterization of 2-Aminooxazolines as Highly Potent, Selective, and Orally Active TAAR1 Agonists. ACS Med. Chem. Lett. 2016; 7(2):192-197). It has also been indicated that these compounds show good oral bioavailability.

The present inventors have found that the compounds of the general Formula I, and in particular the compound of the Formula II, can be more effective in alcoholism treatment than the compounds used for this purpose so far. In particular, these compounds are highly effective in suppressing alcohol relapse (?) after a period of abstinence. The compound of Formula II is additionally easy to prepare and formulate (well soluble in a solution of 5% ethanol and 10% collyphor for an exemplary dose of 10 mg/kg body weight in an intraperitoneal administration), it appears to be non-toxic in animal models.

Preferably, the compound of Formula I is administered in an amount in the range of 1-100 mg/kg, preferably 10-100 mg/kg body weight.

The present inventors have demonstrated efficacy with an exemplary dose of 10 mg/kg body weight in mice. Methods for converting dosages in mice into doses for human administration are known in the art (see, e.g., Nair and Jacob, A simple practice guide for dose conversion between animals and human. J Basic Clin Pharm. 2016; 7(2):27-31).

Preferably, the compound of Formula I is administered up to 24 hours before intended alcohol consumption.

More preferably, the compound of Formula I is administered up to 20 hours before intended alcohol consumption.

More preferably, the compound of Formula I is administered up to 1 hour before intended alcohol consumption.

More preferably, the compound of Formula I is administered less than 1 hour before intended alcohol consumption.

Preferably, the compound of Formula I is administered regularly, preferably every day for a maximum of 3 days, with at least weekly intervals between the 3-day administration periods.

Preferably, the compound of Formula I is administered prior to the intended consumption of beverages with an alcohol content of 20% or more to reduce the motivation to consume high-percentage alcohol.

In the use according to the present invention, the compound of Formula I may be administered to animals, preferably humans, for example before intended alcohol consumption, for example up to 24 hours or more preferably up to 20 hours or particularly preferably 1 hour or even less before intended alcohol consumption, in order to reduce the amount of alcohol consumed.

Alternatively or additionally, the compound of Formula I may be administered to animals, preferably humans, to reduce motivation for alcohol consumption. The compound may be administered once or as needed (e.g., prior to anticipated drinking occasions) or preferably regularly (e.g., every 3, 4, 5 days, every week, or less frequently). Preferably, the compound is administered every day for a maximum period of 3 days. There are intervals of at least one week between these administration periods. Such administration regimen is preferably continued for one month.

Preferably, the compound of Formula I is administered prior to the intended consumption of beverages with an alcohol content of 20% or more, to reduce the motivation to consume high-percentage alcohol. "High-percentage alcohol" is understood as solutions of ethyl alcohol with content of 20% or more thereof.

The compound of Formula I, and in particular the compound of Formula II, is effective in reducing the total amount of alcohol consumed (and this effect lasts for a relatively long time after a single administration, as shown in the examples herein). Additionally, the compound of Formula I, and in particular the compound of Formula II, shows high efficacy in suppressing alcohol consumption after a period of abstinence.

The compound of Formula I, and in particular the compound of Formula II, is also found to be effective in reducing overall motivation to consume alcohol. It can therefore be used for regular administration, e.g. to prevent alcoholism, help reduce alcohol consumption or recover from alcohol dependence, or to treat already developed alcohol use disorder.

The compound of Formula I can be administered in a composition, e.g. in a pharmaceutical preparation, in combination with one or more pharmaceutically acceptable carriers and/or excipients. Pharmaceutically acceptable excipients, carriers, or other additional ingredients mean those which can be reasonably administered to a subject, e.g. a mammal, in particular a human subject, ensuring an effective dose of the active ingredient used.

Pharmaceutical compositions may be formulated for any suitable mode of administration, including, for example, oral or parenteral administration. In some embodiments, compositions in a form suitable for oral use are preferred. Such forms include, for example, pills, tablets, troches, lozenges, aqueous or oily suspensions, dispersible powders or granules, emulsions, hard or soft capsules or syrups or elixirs. The term "parenteral", as used herein, includes subcutaneous, intradermal, intravascular (e.g., intravenous), intramuscular, intraspinal, intracranial, intrathecal and intraperitoneal injection techniques, as well as any similar injection or infusion techniques. Compositions intended for oral use may be prepared by any method known in the art for preparing pharmaceutical compositions, and may contain one or more agents such as flavoring agents, coloring agents, and preservatives. The compositions will usually contain the active ingredient in admixture with physiologically acceptable excipients which are suitable for the manufacture of the given dosage form, e.g. tablets. Such excipients include, for example, inert diluents (e.g., calcium carbonate, sodium carbonate, lactose, calcium phosphate or sodium phosphate), granulating and disintegrating agents (e.g., corn starch or alginic acid), binders (e.g., starch, gelatin or acacia) and lubricants (e.g. magnesium stearate, stearic acid or talc). Tablets and other dosage forms may be uncoated or may be coated by known techniques to delay disintegration and absorption in the gastrointestinal tract and thus provide sustained action over an extended period of time. For example, a delaying material such as glycerol monostearate or glycerol distearate can be used.

The pharmaceutical composition may also be prepared as a sterile injectable aqueous or oil suspension in which the active ingredient, depending on the vehicle and concentration used, is suspended or dissolved in the vehicle. Such a composition may be formulated according to the known art using suitable dispersing, wetting and/or suspending agents such as those mentioned above. Among the acceptable vehicles and solvents that can be used are, for example, water, 1,3-butanediol, Ringer's solution, and isotonic sodium chloride solution. In addition, sterile, fixed oils can be used as a solvent or suspending agent. For this purpose, any colorless solid oil may be employed including synthetic mono- or diglycerides. In addition, fatty acids, such as oleic acid, can be used in the preparation of injectable compositions, and auxiliaries, such as local anesthetics, preservatives and/or buffering agents, can be dissolved in the vehicle.

The pharmaceutical compositions can also, for example, be in the form of an aerosol using a suitable propellant, e.g., dichlorodifluoromethane, trichlorofluoromethane, dichlorotetrafluoroethane, carbon dioxide or other suitable gas. The aerosolized compositions may be for, e.g., oral, but also to the respiratory system (e.g., nasal, pulmonary) administration.

The pharmaceutical compositions can be formulated as sustained release formulations (i.e., a formulation such as a capsule which causes a slow release of the active ingredient after administration). Such formulations can generally be prepared using well-known technology and administered, for example, by oral administration or by application at the desired target site. The carriers for use in such formulations are biocompatible and may also be biodegradable; preferably the formulation provides a relatively constant level of active ingredient release. The amount of active ingredient contained in the sustained release formulation depends, for example, on the site of insertion or implantation, the rate and expected duration of release, and the nature of the disease/condition being treated.

A pharmaceutical composition containing a compound of Formula I for use according to the invention may be in the form of, for example, a kit comprising a composition container and/or, for example, unit dosage forms, as well as a label and instructions for use.

### Brief Description of the Figures

Fig. 1 shows the IntelliCage used in the experiments. 1 - food holder; 2 - bottle for water or alcohol, 3 - mouse habitat.
Fig. 2 shows the average amount of consumed alcohol before the start of the experiment.
Fig. 3 shows the average amount of consumed alcohol during 20 hours after injection by the control group (vehicle) and RO5256390 group; graph for 5 animals from each group that had the highest alcohol consumption before the start of the experiment.
Fig. 4 shows the average amount of consumed alcohol in the 20 hours post injection by the control group (vehicle) and RO5256390 group, after a 10 day abstinence period; graph for 5 animals from each group that characterized in the highest alcohol consumption before the start of the experiment.
Fig. 5 shows the mean number of alcohol drinking attempts measured before injection and after administration of vehicle or vehicle and trace amine-associated receptor 1 agonist (RO5256390).

### EXAMPLES

### Example 1: The effect on the amount of alcohol consumed.

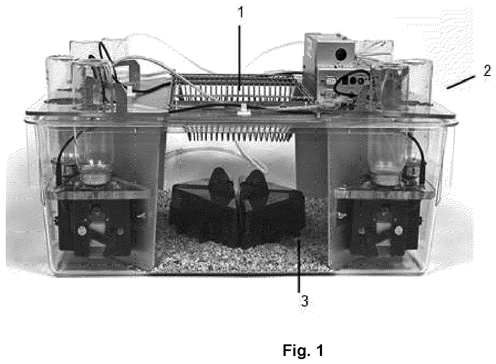

The study involved 30 C57BL/6 mouse subjects that had undergone a two-month, earlier adaptive training to drink alcohol (20%) in IntelliCages (Fig. 1), under typical living conditions (temperature: **21-23°C, humidity 50-60%,** 12/12 light-dark cycle). During the adaptive training, the mice had constant access to food and water except for:
1. 25 days, during which, 2 hours after the beginning of the dark phase, all water bottles were replaced for 3 hours with bottles containing 20% alcohol (no access to clean water at all).
2. 15 days, during which, 2 hours after the beginning of the dark phase, half of the water bottles were replaced for 3 hours with bottles containing 20% alcohol (possibility to choose water or alcohol).

Contrary to previous studies, modern, fully automated IntelliCages were used here, which allow for:
- keeping animals in a larger group, which imitates natural social behavior,
- minimal involvement of the experimenter during the study,
- behavior tracking and identification of particular animals by using subcutaneous transponders,
- conducting sophisticated tests, incl. assessing the motivation to drink alcohol.

During the adaptive training, the amount of alcohol consumed was recorded for each animal separately. Based on their preference for drinking alcohol, the mice were divided into two experimental groups (vehicle/control and RO5256390), each of which included animals characterized by high, medium and low alcohol consumption. The degree of preference for drinking alcohol was determined according to the following criteria:
- high-drinkers: on average from 100 to 200 licks of alcohol, with a choice between water and alcohol (3 hour session);
- medium-drinkers: on average from 50 to 100 licks of alcohol, with a choice between water and alcohol; (3 hour session);
- low-drinkers: on average from 0 to 50 licks of alcohol, with a choice between water and alcohol; (3 hour session);

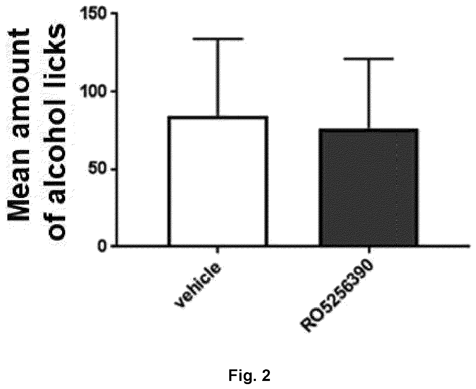

The average amount of consumed alcohol before the start of the experiment was similar for both groups (Fig. 2).

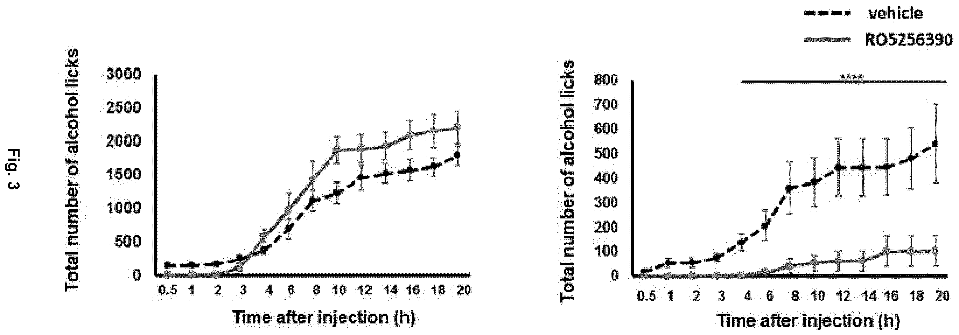

Then, prior to the administration of alcohol, the mice received intraperitoneal injections, during which they were given a vehicle in the form of 1 part pure alcohol, 2 parts collifor and 17 parts saline (vehicle group - control group) or a vehicle with a trace amine-associated receptor 1 agonist dissolved therein, RO5256390 ("RO5256390" group), at a dose of 10 mg/kg body weight (bw). The amount of consumed alcohol was measured during 20 hours after injection. The animals treated with R05256390 consumed a statistically significant lower amount of alcohol than the animals in the control group (Fig. 3), with a concomitant, similar amount of water.

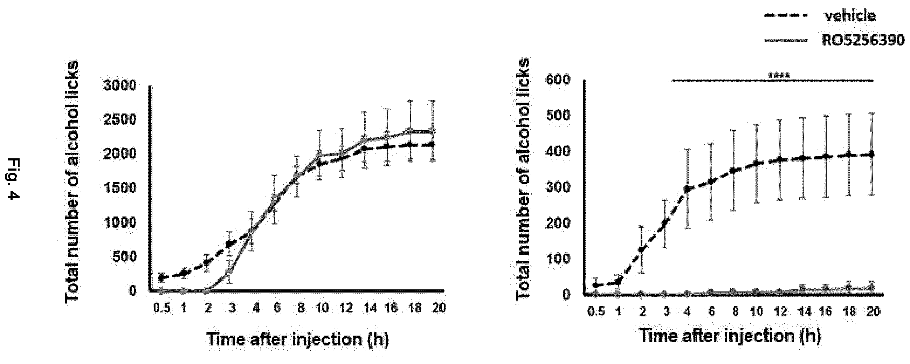

At a later stage of the study, the access to alcohol was blocked for 10 days. Before the access to alcohol was restored, the animals were treated as previously with a vehicle or vehicle with a trace amine-associated receptor 1 agonist, RO5256390 (RO5256390) dissolved therein. The animals treated with R05256390 consumed statistically significantly lower amount of alcohol than the animals from the control group (Fig. 4), with a concomitant, similar amount of water drunk.

### Example 2: Influence on motivation to drink alcohol.

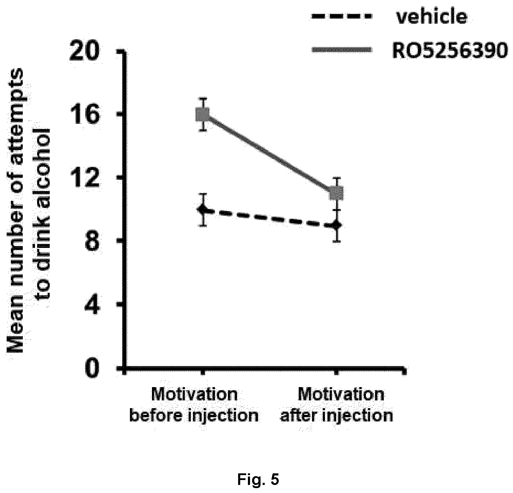

The effect of RO5256390 administration on motivation to drink alcohol was also assessed experimentally. In this experiment a new group of animals was used, these were 13 animals that underwent a three-month adaptive training to drink alcohol (20%) in IntelliCages. The motivation to drink alcohol was then assessed for each animal by conducting 3 tests. For the purposes of the test, the IntelliCage was designed in such a way that access to alcohol for a given animal was only possible, with a certain number of so-called "nose-pokes", meaning cutting the laser beam with the snout when trying to reach the alcohol bottle. In order to drink alcohol, the animal must first cut the beam twice, then 4, 8, 12, 16 times etc. After the required number of nose-pokes are made, the alcohol-blocking door opens. The more attempts to get into the alcohol bottle and the greater the number of beam intersection events achieved, the greater the motivation to drink alcohol. Each test started when half of the water bottles was replaced with 20% alcohol bottles. During the next 20 hours the number of drinking attempts was measured for each animal. After 3 series of motivational tests, which were carried out at weekly intervals, the animals were divided into two experimental groups. The animals with the highest score in the motivational tests were assigned to the experimental group (RO5256390), while the animals with worse result were assigned to the control group (RO5256390). After the mice were allocated to the groups, 3 motivation tests were performed again, this time preceded by the intraperitoneal administration of a vehicle or a trace amine-associated receptor 1 agonist (RO5256390) at a dose of 10 mg/kg bw for 10 min. On the basis of the conducted experiments, it was found that the intraperitoneal administration of RO5256390 reduces the motivation to drink alcohol (Fig. 5).

## Claims

1. 4,5-dihydro-oxazol-2-ylamine derivatives of general Formula I: 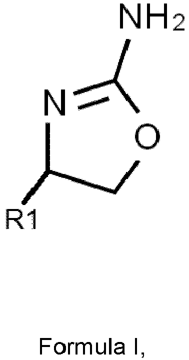 wherein R1 denotes:
- straight or branched (C1-C4) alkyl, optionally substituted, including in particular (C1-C4) alkyl substituted with a phenyl group, or
- phenyl, optionally substituted with one or more halogen, or
- 2-[(N-(C1-C2)alkyl-N-aryl)amin-(C1-C2)alkyl]- group
for use in a method of treating or preventing alcohol use disorder by reducing alcohol consumption and/or motivation to drink alcohol, wherein the compound of Formula I is administered prior to intended alcohol consumption to reduce motivation to drink alcohol.

2. 4,5-dihydro-oxazol-2-ylamine derivatives for use according to claim 1, wherein the compound of general Formula I is a compound of Formula II:

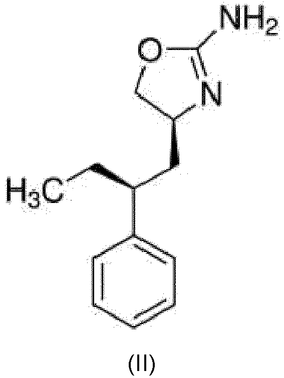

3. 4,5-dihydro-oxazol-2-ylamine derivatives for use according to claim 1, **characterized in that** the compound of Formula I is administered in an amount in the range of 1-100 mg/kg body weight, preferably 10-100 mg/kg body weight.

4. 4,5-dihydro-oxazol-2-ylamine derivatives for use according to any one of claims 1-3, **characterized in that** the compound of Formula I is administered up to 24 hours before the intended alcohol consumption.

5. 4,5-dihydro-oxazol-2-ylamine derivatives for use according to claim 4, **characterized in that** the compound of Formula I is administered up to 20 hours before the intended alcohol consumption.

6. 4,5-dihydro-oxazol-2-ylamine derivatives for use according to claim 4, **characterized in that** the compound of Formula I is administered up to 1 hour before the intended alcohol consumption.

7. 4,5-dihydro-oxazol-2-ylamine derivatives for use according to claim 4, **characterized in that** the compound of Formula I is administered less than 1 hour prior to the intended alcohol consumption.

8. 4,5-dihydro-oxazol-2-ylamine derivatives for use according to any one of claims 1-7, **characterized in that** the compound of Formula I is administered regularly, preferably every day for a maximum period of 3 days, with at least weekly intervals between the 3-day administration periods.

9. 4,5-dihydro-oxazol-2-ylamine derivatives for use according to any one of claims 1-8, **characterized in that** the compound of Formula I is administered before the intended consumption of beverages with an alcohol content of 20% or more, in order to reduce the motivation to consume high-percentage alcohol.
